# EUROPÄISCHE PATENTSCHRIFT

(11) **EP 2 416 714 B1**
(45) Veröffentlichungstag und Bekanntmachung des Hinweises auf die Patenterteilung: **08.05.2019**
(21) Anmeldenummer: 10708929.4
(22) Anmeldetag: 10.03.2010
(51) Int. Cl.: A61B 17/3203, B05B 1/34

(54) **WASSERSTRAHLCHIRURGIEINSTRUMENT**
WATER JET SURGICAL INSTRUMENT
INSTRUMENT CHIRURGICAL À JET D'EAU

(30) Priorität: 08.04.2009 DE 102009016859
(43) Veröffentlichungstag der Anmeldung: 15.02.2012
(73) Patentinhaber: Erbe Elektromedizin GmbH, 72072 Tübingen (DE)
(72) Erfinder: KARWEI, Dietmar, 72131 Ofterdingen (DE)
(74) Vertreter: Rüger Abel
(86) Internationale Anmeldenummer: PCT/EP2010/001489
(87) Internationale Veröffentlichungsnummer: WO 2010/115499

(56) Entgegenhaltungen:
- EP-A1- 1 800 612
- DE-A1-102007 002 486
- DE-T2- 69 824 728
- US-A- 5 674 226
- US-A- 5 865 790
- US-A1- 2002 007 143

## Beschreibung

Die Erfindung betrifft ein Wasserstrahlchirurgieinstrument nach dem Oberbegriff des Patentanspruches 1.

Wasserstrahlchirurgievorrichtungen finden zunehmend in der Chirurgie Anwendung, weil ihr Schneid-, oder besser gesagt, ihr Trennverhalten sich von dem üblicher Skalpelle sowie alternativer Vorrichtungen wie Laserchirurgie- oder Hochfrequenzchirurgiegeräten unterscheidet.

Insbesondere sind mit Wasserstrahlchirurgievorrichtungen Schnitte möglich, die bestimmte Gewebearten durchtrennen und andere unangetastet lassen (selektive Gewebetrennung).

Solche Techniken finden schließlich zunehmend Anwendung bei endoskopischen Verfahren für die Chirurgie.

Derartige Wasserstrahlchirurgievorrichtungen umfassen ein Fluidreservoir und eine Fördereinrichtung zur Förderung des Fluid durch eine Druckleitung vom Fluidreservoir zu einem an der Wasserstrahlchirurgievorrichtung angeschlossenen Wasserstrahlchirurgieinstrument. Das Wasserstrahlchirurgieinstrument weist seinerseits eine Ausstoßdüse auf, um das Fluid in Form eines feinen Fluidstrahls auszustoßen.

Bei der Resektion von Gewebe, z.B. Tumorgewebe im Gastrointestinaltrakt, muss der Wasserstrahl mit hohem Druck gebündelt aus der Ausstoßdüse austreten. Wenn - wie im genannten Anwendungsbeispiel - die Resektion des Tumorgewebes auf die Mukosa begrenzt ist, soll das Tumorgewebe möglichst in einer Sitzung und möglichst vollständig ektomiert werden, wie dies mit einem Fluidstrahl möglich ist. Problematisch ist dabei aber, dass der "gebündelte" Fluidstrahl zu einer Perforation der *muskularis propria* führen kann. Die Folge sind gefährliche innere Blutungen, wobei der OP-Situs verdeckt wird. Für andere Anwendungen von Wasserstrahlchirurgievorrichtungen ist es wiederum wünschenswert, den Fluidstrahl in seinem Austrittswinkel so zu verändern, dass unabsichtliche Verletzungen durch den Operateur unterbleiben bzw. andere Funktionen des Fluidstrahls ermöglicht werden (z.B. Spülen).

Aus der US 5 944 686 ist ein chirurgisches Instrument für die Wasserstrahlchirurgie bekannt, bei welchem ein schneidender Fluidstrahl über eine Ablenkfläche zerstäubt bzw. in seinem Austrittswinkel verändert wird. Diese Ablenkfläche ist dabei distal von der eigentlichen Ausstoßdüse angeordnet.

Aus der DE 10 2007 002 486 A1 ist ein Wasserstrahlchirurgieinstrument bekannt, bei dem ein an sich im Querschnitt runder Fluidstrahl auf eine löffelartige Prallfläche triff und dort in einen abgeflachten Wasserstrahl verwandelt wird. Diese löffelartige Fläche ist dabei als verlängertes distales Ende eines hier beginnenden Absaugrohrs ausgebildet, ist also wieder distal van der Ausstoßdüse angeordnet.

Übereinstimmend wird also bisher vorgeschlagen, besondere Vorrichtungen wie Prall- oder Ablenkflächen vorzusehen, um dem Fluidstrahl eine gegenüber einem vom runden Querschnitt abweichende Form bzw. einen anderen Austrittswinkel zu geben. Derartige Zusatzeinrichtungen stören aber die freie Sicht auf den OP-Situs sowie das Arbeiten mit dem Instrument selbst, da dem eigentlichen Fluidaustrittselement, der Ausstoßdüse, immer eine weitere Vorrichtung nachgeschaltet ist und dies an einer für das chirurgische Arbeiten ungünstigen Stelle.

Aus der DE 69824728 T2 ist darüber hinaus ein pneumatischer Gewebedissektor mit Druckentlastungssystem bekannt, der zum Trennen von biologischem Gewebe einen Gasstrahl nutzt. Dieser wird durch eine Düse ausgestoßen, der ein Drosselglied vorgelagert ist. Das Drosselglied kann durch Verdrehung mittels eines Gewindes entlang der Längsachse des Instruments verstellt werden, umso näher an die Ausströmdüse heran oder etwas weiter von dieser weg bewegt zu werden. Dadurch kann der Gasstrom je nach Position des Drosselglieds weniger konzentriert und langsamer oder konzentriert und schneller ausgestoßen werden. Ein breites Gasmuster soll für die stumpfe Dissektion besser geeignet sein, wohingegen ein konzentrierter Gasstrom für präziseres Schneiden von Gewebe empfohlen wird.

Die US 2002/0007143 A1 offenbart ein Verfahren zur Erzeugung eines gepulsten Microjets aus eine Hydraulikfluid. Dazu ist in einer Hydraulikkammer eine flexible Pumpkammer angeordnet, die unter äußeren Hydraulikdruck kollabiert, um eine Fluidportion auszustoßen. Fortwährende Wiederholung dieses Vorgangs führt zu einem gepulsten Strahl.

Die US-A-5674226 offenbart eine Vorrichtung zur Erzeugung eines pulsierenden Fluidstrahls. Der Erfindung liegt die Aufgabe zu Grunde, ein Wasserstrahlchirurgieinstruments aufzuzeigen, das in konstruktiv einfacher Weise eine Steuerung der Geometrie des Fluidstrahls ermöglicht, ohne die Handhabbarkeit des Instrumentes wesentlich zu verschlechtern.

Diese Aufgabe wird durch ein Wasserstrahlchirurgieinstrument nach Anspruch 1 gelöst.

Insbesondere wird die Aufgabe durch ein Wasserstrahlchirurgieinstrument gelöst, umfassend
- eine Zuführleitung zum Zuführen eines Fluids in einer Strömungsrichtung,
- eine Austrittsdüse zum Ausstoßen des Fluids in einem Strahl mit definierter Öffnungsgeometrie, wobei in Strömungsrichtung des Fluids vor der Austrittsdüse eine Fluidkammer vorgesehen ist und wobei in der oder an der Fluidkammer eine Störvorrichtung vorgesehen ist, durch welche eine turbulente Strömung in der Fluidkammer erzeugbar ist.

Die Strömung des Fluids ist in der ausreichend groß dimensionierten und vorzugsweise auch symmetrisch gebauten Fluidkammer zunächst laminar. Das heißt, dass in der Strömung die Trägheitskräfte an jeder Stelle der Fluidkammer gegenüber den Reibungskräften überwiegen. Erreicht das Fluid einer solchen Strömungsform die Ausstoßdüse, so wird ein mehr oder weniger feiner, jedenfalls beim Austritt scharf umrissener Fluidstrahl aus der Ausstoßdüse austreten - natürlich vorausgesetzt, dass die Düsengeometrie entsprechend aufgebaut ist.

Überraschenderweise hat es sich nun gezeigt, dass sich die Form des austretenden Fluidstrahls dahin gehend ändert, dass der Fluidstrahl nicht mehr als feiner Strahl austritt, sobald die Strömung in der Fluidkammer beginnt, turbulent zu werden. Der Fluidstrahl ist dann mehr oder weniger aufgeweitet. Dies ist insofern besonders überraschend, als gar nicht in den eigentlichen Fluidstrahl nach dem Verlassen der Ausstoßdüse eingegriffen wird, sondern die Strömung bei vollkommen unveränderter Situation an oder nach Verlassen der Ausstoßdüse viel weiter davor in der Fluidkammer verändert wird. Dadurch aber ergibt sich die Möglichkeit, das Strömungsverhalten an einer Stelle der Strömung zu verändern, die für den Anwender des Wasserstrahlchirurgieinstruments viel weniger störend ist.

Um die Strömung in der Fluidkammer turbulent werden zu lassen, wird die Geometrie der Fluidkammer an irgendeiner Stelle so verändert, dass eine Asymmetrie bezogen auf die Länge, die Breite und/oder den Radius in der Strömung auftritt. Dadurch wird die Strömung dort stellenweise turbulent. Hier überwiegen dann die Reibungskräfte und die Trägheitskräfte der Moleküle.

Bei einer ersten bevorzugten Ausführungsform umfasst die Störvorrichtung zur Umwandlung der laminaren Strömung in eine turbulente Strömung einen umströmbaren oder durchströmbaren Körper, der in der Fluidkammer angeordnet ist. Bei ansonsten gleich bleibender Geometrie der Fluidkammer hat es sich herausgestellt, dass eine ortsfeste Störvorrichtung zu einer gleich bleibenden und wiederholbaren Strahlaufweitung des Fluidstrahls führt.

Bei einer anderen bevorzugten Ausführungsform der Erfindung umfasst die Störvorrichtung eine vorzugsweise flexible Wand der Fluidkammer. Durch die Einstellung der Form (des Querschnitts) der Fluidkammer kann somit ebenfalls eine reproduzierbare Strahlaufweitung bewerkstelligt werden.

Die Störvorrichtung kann fest in die Fluidkammer eingebaut sein bzw. die Fluidkammer kann unveränderbar dimensioniert sein, wobei man dann für verschiedene Strahlgeometrien verschiedene Wasserstrahlchirurgieinstrumente bereitstellen kann.

Bei einer bevorzugten Ausführungsform der Erfindung wird die Störvorrichtung zur Änderung der Öffnungsgeometrie des Strahls hinsichtlich ihrer Position und/oder Geometrie einstellbar ausgebildet. Der Operateur kann dann den Fluidstrahl durch eine entsprechende Einstellung der Störvorrichtung so "formen", dass er seinen Zwecken genügt. Dies kann beispielsweise durch eine Betätigungseinrichtung zum Verschieben der Störvorrichtung in der Fluidkammer geschehen. Die Betätigungseinrichtung umfasst bei der bevorzugten Ausführungsform der Erfindung ein Zug- und Schubelement, insbesondere einen Draht zum Verschieben der Störvorrichtung in der Fluidkammer. Dieses Zug- und Schubelement ist dann mit einem vom Operateur betätigbaren Handgriff verbunden.

Alternativ kann die Betätigungseinrichtung auch ein Magnetelement zum Aufbringen einer Magnetkraft auf die Störvorrichtung umfassen. In diesem Fall ist ein mechanischer Eingriff in die Fluidkammer nicht notwendig, so dass die bei den hohen Drücken nicht unproblematische Abdichtung der Betätigungseinrichtung entfällt.

Vorzugsweise wird das Wasserstrahlchirurgieinstrument zum Einsetzen in einen Arbeitskanal eines Endoskops ausgebildet. Hier zeigen sich die Vorteile des Instrumentes besonders drastisch dann, wenn die Störvorrichtung vom Operateur veränderbar ist. Ein Instrumentenwechsel ist in diesem Fall nämlich besonders aufwändig, muss aber bei der Einstellbarkeit der Störvorrichtung nicht mehr vorgenommen werden.

Nachfolgend werden bevorzugte Ausführungsformen der Erfindung anhand von Abbildungen näher erläutert. Hierbei zeigen
- - Fig. 1: eine Wasserstrahlchirurgievorrichtung in einer schematisierten Darstellung mit einer ersten Position einer Störvorrichtung,
- - Fig. 2: die hier wesentlichen Teile des Wasserstrahlchirurgieinstruments nach Fig. 1 mit einer hierzu veränderten Position der Störvorrichtung,
- - Fig. 3: eine weitere Ausführungsform der Erfindung basierend auf einer Störvorrichtung gemäß Fig. 1 oder Fig. 2 und
- - Fig. 4: eine weitere Ausführungsform der Erfindung mit zwei Positionen der Störvorrichtung in den Zeichnungsteilen A und B.

In der nachfolgenden Beschreibung werden für gleiche und gleich wirkende Teile dieselben Bezugsziffern verwendet.

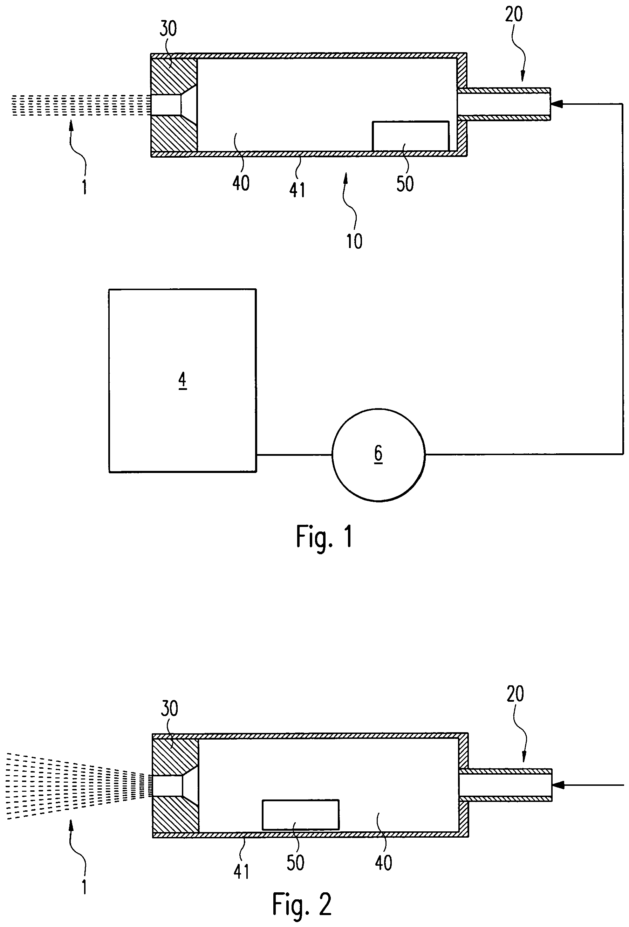

In Fig. 1 ist eine Ausführungsform der Erfindung gezeigt, bei welcher ein Wasserstrahlchirurgieinstrument 10 über eine Zuführleitung 20 und eine Pumpe 6 mit einem Fluidreservoir 4 verbunden ist. Zwischen der Zuführleitung 20 und einer Ausstoßdüse 30 ist eine Fluidkammer 40 vorgesehen, die eine Außenwand aufweist. Die Fluidkammer 40 ist hierbei derart bemessen, dass sich in ihr eine im Wesentlichen laminare Strömung des Fluids aus dem Fluidreservoir 4 ausbildet, wenn dieses durch die Ausstoßdüse 30 ausgestoßen wird. Hierbei ist die Ausstoßdüse 30 derart geformt, dass sich bei einer laminaren Strömung in der Fluidkammer 40 ein im Wesentlichen "zylindrischer" Fluidstrahl 1 ausbildet. Mit einem derartigen Fluidstrahl 1 kann bei entsprechend hohem Druck die gewünschte Behandlung von Gewebe, nämlich ein selektives Schneiden desselben durchgeführt werden.

In der Fluidkammer 40 ist eine Störvorrichtung 50 angeordnet, die in dem in Fig. 1 schematisiert dargestellten Ausführungsbeispiel als kantiger Körper ausgebildet ist, der dann, wenn er - wie in Fig. 2 gezeigt - in Richtung auf die Ausstoßdüse 30 verschoben wird, die zunächst laminare Strömung in der Fluidkammer 40 dadurch in eine turbulente Strömung verwandelt, dass der kantige Körper vom Fluidstrom umströmt und dieser an seinen Kanten "gebrochen" wird. Wenn die in Fig. 2 gezeigte Situation vorliegt, so liegt also in der Fluidkammer 40 kurz vor der Ausstoßdüse 30 eine turbulente Strömung vor. Durch diese turbulente Strömung wird nun der Fluidstrahl 1 aufgeweitet, wie dies in Fig. 2 gezeigt ist. Allein dadurch also, dass die Art der Strömung (laminar/turbulent) vor der Ausstoßdüse 30 verändert wird, kann die Geometrie des Fluidstrahls 1 beeinflusst werden. Somit kann dem Chirurgen ein Set von Wasserstrahlchirurgieinstrumenten 10 zur Verfügung gestellt werden, bei welchen die Störvorrichtung 50 an verschiedenen Stellen (oder in verschiedenen Formen) in der Fluidkammer 40 angebracht ist. Demzufolge hat dann der Fluidstrahl 1 die eine oder andere geometrische Form, also gebündelt oder aufgefächert.

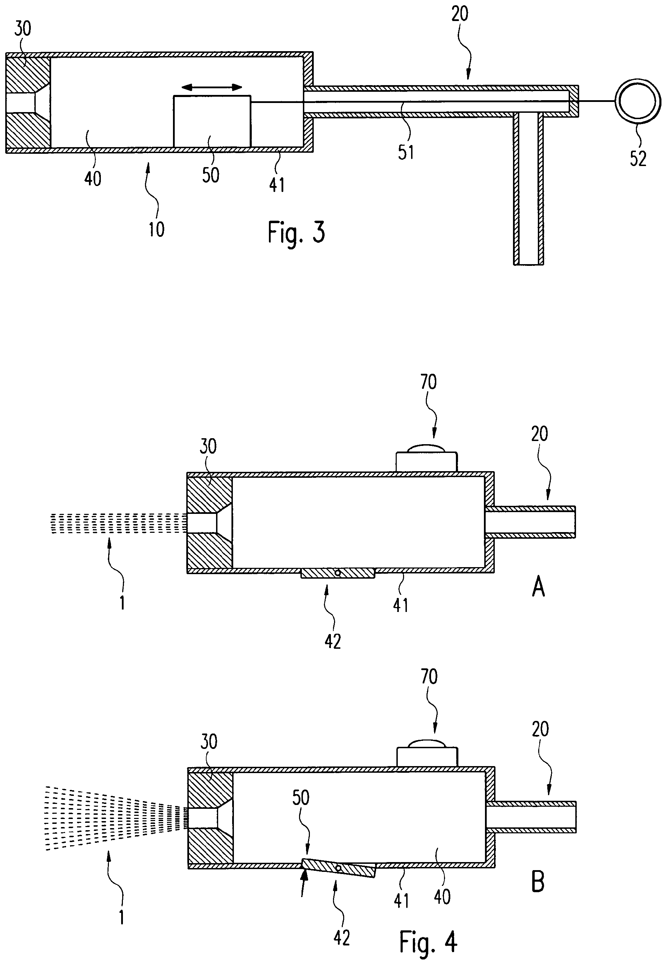

Die in Fig. 3 gezeigte Ausführungsform der Erfindung unterscheidet sich von derjenigen nach den Fig. 1 und 2 dadurch, dass die Störvorrichtung 50 - wie mit dem Doppelpfeil angedeutet - innerhalb der Fluidkammer 40 verschoben werden kann. Hierzu ist ein Zug- und Schubelement 51 vorgesehen, das als steifer Draht ausgebildet sein kann. Dieses Zug- und Schubelement 51 ist aus der Zuführleitung 20 über eine Dichtung ausgeführt und weist an seinem Ende einen ersten Handgriff 52 auf. Über diesen Handgriff 52 kann somit der Operateur während der Operation die geometrische Form des Fluidstrahles 1 einstellen, indem er die Störvorrichtung 50 verschiebt. Diese Möglichkeit ist nicht nur in der offenen Chirurgie von Vorteil, sie bietet einen besonderen Vorteil vielmehr auch in der endoskopischen Anwendung, da dort ein Instrumentenwechsel zum Ersetzen eines gebündelten durch einen aufgeweiteten Strahl besonders vorteilhaft ist.

Bei der in Fig. 4 gezeigten Ausführungsform ist die Störvorrichtung 50 dadurch gebildet, dass die Außenwand 41 der Kammer 40 einen beweglichen Außenwandabschnitt 42 aufweist. Wenn dieser verändert wird, sich also die Geometrie der Fluidkammer 40 ändert, so kann ebenfalls eine turbulente Strömung innerhalb der Fluidkammer 40 und damit eine Aufweitung des Fluidstrahls 1 erzeugt werden. Dies ist in Fig. 4 in den Beispielen A (laminare Strömung = gebündelter Strahl) und B (turbulente Strömung = aufgeweiteter Fluidstrahl) gezeigt. Dieser bewegliche Außenwandabschnitt 42, der also die Störvorrichtung 50 bildet, ist nun so gelagert, dass bei einem Verkippen (siehe Pfeilrichtung in Fig. 4B) ein Teil des Außenwandabschnitts 42 in die Fluidkammer 40 eindringt, während ein anderer Teil des beweglichen Außenwandabschnitts 42 weiter aus der Fluidkammer 40 herauskippt. Dadurch wird eine Neutralisierung der Kräfte erreicht, die aufgrund des Fluiddrucks 40 innerhalb der Kammer auf den Außenwandabschnitt 42 wirken.

Zusätzlich zu dem beweglichen Außenwandabschnitt 42 als Betätigungseinrichtung ist noch ein Aktivierungsschalter 70 vorgesehen, so dass man sich das in Fig. 4 gezeigte Wasserstrahlchirurgieinstrument insgesamt als handhabbares Teil für die offene Chirurgie vorstellen kann.

Aus Obigem geht hervor, dass die Erfindung auch ein Verfahren betrifft, um bei einem Wasserstrahlchirurgieinstrument den austretenden Fluidstrahl in seiner Form zu verändern, indem man eine vor der Ausstoßdüse anstehende laminare Strömung in eine turbulente Strömung verwandelt.

### Bezugszeichenliste

- 1: Fluidstrahl
- 4: Fluidreservoir
- 6: Pumpe
- 10: Wasserstrahlchirurgieinstrument
- 20: Zuführleitung
- 30: Ausstoßdüse
- 40: Fluidkammer
- 41: Außenwand der Fluidkammer
- 42: beweglicher Außenwandabschnitt
- 50: Störvorrichtung
- 51: Zug- und Schubelement
- 52: Handgriff für Zug- und Schubelement
- 70: Aktivierungsschalter für Wasserstrahlchirurgievorrichtung

## Patentansprüche

1. Wasserstrahlchirurgieinstrument, umfassend
- eine Zuführleitung (20) zum Zuführen eines Fluids in einer Strömungsrichtung;
- eine Ausstoßdüse (30) zum Ausstoßen des Fluids in einem Strahl mit einer definierten Öffnungsgeometrie,
wobei
in Strömungsrichtung des Fluids vor der Ausstoßdüse (30) eine Fluidkammer (40) vorgesehen ist und in oder an der Fluidkammer (40) eine Störvorrichtung (50) vorgesehen ist, durch welche eine turbulente Strömung in der Fluidkammer (40) erzeugbar ist, **dadurch gekennzeichnet, dass** die Störvorrichtung einen verkippbaren Außenwandabschnitt (42) der Fluidkammer (40) umfasst, der beim Verkippen derart in die Fluidkammer eindringt, dass eine turbulente Strömung erzeugbar ist.

2. Wasserstrahlchirurgieinstrument nach Anspruch 1, **dadurch gekennzeichnet, dass** die Störvorrichtung (50) einen um- oder durchströmbaren Körper (50) umfasst.

3. Wasserstrahlchirurgieinstrument nach einem der vorhergehenden Ansprüche, **dadurch gekennzeichnet dass** die Störvorrichtung (50) zur Veränderung der Öffnungsgeometrie des Fluidstrahls (1) einstellbar hinsichtlich ihrer Position und/oder Geometrie ausgebildet ist.

4. Wasserstrahlchirurgieinstrument nach einem der vorhergehenden Ansprüche, insbesondere nach Anspruch 4, **gekennzeichnet durch** eine Betätigungseinrichtung (51) zum Verschieben der Störvorrichtung (50) in der Fluidkammer (40).

5. Wasserstrahlchirurgieeinrichtung nach einem der vorhergehenden Ansprüche, insbesondere nach Anspruch 4, **dadurch gekennzeichnet, dass** die Betätigungseinrichtung ein Zug- und Schubelement (51), insbesondere einen Draht zum Verschieben der Störvorrichtung (50) in der Fluidkammer (40) umfasst.

6. Wasserstrahlchirurgieinstrument nach einem der vorhergehenden Ansprüche, insbesondere nach einem der Ansprüche 4 oder 5, **dadurch gekennzeichnet dass** die Betätigungseinrichtung ein Magnetelement zum Aufbringen einer Magnetkraft auf die Störvorrichtung (50) umfasst.

7. Wasserstrahlchirurgieinstrument nach einem der vorhergehenden Ansprüche, **dadurch gekennzeichnet dass** es zum Einsetzen in einen Arbeitskanal eines Endoskops ausgebildet ist.

## Claims

1. Water jet surgical instrument, comprising:
- a supply line (20) for supplying a fluid in a flow direction,
- an outlet nozzle (30) for ejecting the fluid in a jet with a defined opening geometry,
wherein upstream of the outlet nozzle (30) in the flow direction of the fluid, a fluid chamber (40) is provided, in or on which fluid chamber (40) a disruption device (50) is provided which can create a turbulent flow in the fluid chamber (40), **characterised in that** the disruption device comprises a tiltable outer wall portion (42) of the fluid chamber (40) which when tilted penetrates into the fluid chamber such that a turbulent flow can be generated.

2. Water jet surgical instrument according to claim 1, **characterised in that** the disruption device (50) comprises a body (50) around or through which fluid may flow.

3. Water jet surgical instrument according to any of the preceding claims, **characterised in that** the disruption device (50) is configured so as to be adjustable in its position and/or geometry in order to change the opening geometry of the fluid jet (1).

4. Water jet surgical instrument according to any of the preceding claims, in particular claim 4¹, **characterised by** an actuation device (51) for moving the disruption device (50) in the fluid chamber (40).

5. Water jet surgical instrument according to any of the preceding claims, in particular claim 4, **characterised in that** the actuation device comprises a push and pull element (51), in particular a wire for moving the disruption device (50) in the fluid chamber (40).

6. Water jet surgical instrument according to any of the preceding claims, in particular one of claims 4 or 5, **characterised in that** the actuation device comprises a magnet element for applying a magnetic force to the disruption device (50).
¹*Translator's note: Clearly an incorrect back reference.*

7. Water jet surgical instrument according to any of the preceding claims, **characterised in that** it is configured for insertion in a working channel of an endoscope.

## Revendications

1. Instrument chirurgical à jet d'eau, comprenant
- une conduite d'alimentation (20) destinée à amener un fluide dans un sens d'écoulement ;
- une buse d'éjection (30) destinée à éjecter le fluide en un jet avec une géométrie d'ouverture définie ;
dans lequel
une chambre à fluide (40) est prévue en amont de la buse d'éjection (30), dans le sens d'écoulement du fluide, et un dispositif de perturbation (50) est prévu dans ou sur la chambre à fluide (40) et permet de générer un écoulement turbulent dans la chambre à fluide (40), **caractérisé en ce que** le dispositif de perturbation comprend une partie de paroi extérieure (42) inclinable de la chambre à fluide (40) qui, lorsqu'elle est inclinée, pénètre dans la chambre à fluide (40) de manière telle qu'il permette de générer un écoulement turbulent.

2. Instrument chirurgical à jet d'eau selon la revendication 1, **caractérisé en ce que** le dispositif de perturbation (50) comprend un corps (50) qui peut être entouré ou traversé par l'écoulement.

3. Instrument chirurgical à jet d'eau selon l'une des revendications précédentes, **caractérisé en ce que**, pour la modification de la géométrie d'ouverture du jet de fluide (1), le dispositif de perturbation (50) est réalisé de manière à pouvoir être réglé quant à sa position et/ou sa géométrie.

4. Instrument chirurgical à jet d'eau selon l'une des revendications précédentes, notamment selon la revendication 4,**caractérisé en ce qu'**il comporte un dispositif d'actionnement (51) destiné à déplacer le dispositif de perturbation (50) dans la chambre à fluide (40).

5. Dispositif chirurgical à jet d'eau selon l'une des revendications précédentes, notamment selon la revendication 4, **caractérisé en ce que** le dispositif d'actionnement comprend un élément de traction et de poussée (51), en particulier un fil métallique pour déplacer le dispositif de perturbation (50) dans la chambre à fluide (40).

6. Instrument chirurgical à jet d'eau selon l'une des revendications précédentes, notamment selon l'une des revendications 4 ou 5, **caractérisé en ce que** le dispositif d'actionnement comprend un élément magnétique pour appliquer une force magnétique au dispositif de perturbation (50).

7. Instrument chirurgical à jet d'eau selon l'une des revendications précédentes, **caractérisé en ce qu'**il est conçu en vue d'une insertion dans un canal de travail d'un endoscope.
